# EUROPEAN PATENT SPECIFICATION

(11) **EP 1 896 090 B1**
(45) Date of publication and mention of the grant of the patent: **02.09.2009**
(21) Application number: 05755050.1
(22) Date of filing: 29.06.2005
(51) Int. Cl.: A61L 29/14, A61M 25/16, A61N 1/05, C08J 7/14

(54) **SURFACE MODIFICATION OF IMPLANTABLE ARTICLE**
OBERFLÄCHENMODIFIZIERUNG EINES IMPLANTIERBAREN ARTIKELS
MODIFICATION DE SURFACE D'ARTICLE IMPLANTABLE

(43) Date of publication of application: 12.03.2008
(73) Proprietor: St. Jude Medical AB, 175 84 Järfälla (SE)
(72) Inventor: DOWLING, Kenneth, S-197 91 Bro (SE)
(86) International application number: PCT/SE2005/001029
(87) International publication number: WO 2007/001218

(56) References cited:
- US-A- 4 836 884
- US-A- 4 882 148
- US-A1- 2002 018 898
- US-A1- 2005 021 002
- US-B1- 6 438 425

## Description

### FIELD OF THE INVENTION

This invention relates to modification of the surface characteristics of at least one surface of an elastomeric article, for example tubing, composed of polymeric materials such as silicone rubber, polypropylene, polyethylene, polyvinylchloride, fluoropolymers and the like or other dielectric materials and to an improved method for effecting such modifications. The invention also concerns a method of assembly of an implantable electrode lead.

### BACKGROUND OF THE INVENTION

Polymeric plastic tubing, particularly that of small diameter, and most especially that of silicone rubber, is used in many medical applications and devices. Actually, silicone rubber (especially peroxide cross linked silicone elastomer with silica filling) is the polymer of choice for tubing in many medical applications involving implantation. In many instances this tubing is less than about 2 mm in inner diameter (ID).

Although this invention is applicable to other polymeric materials and dielectric materials, it will be described herein with particular reference to silicone rubber, the preferred embodiment. For example, the so called "screw-in" pacing leads make use of very small diameter tubing such as less than 1.4 mm outer diameter (OD) with an ID of 0.9 mm. In this type of lead, an elongate wire core (usually in the form of a coil) having a helical screw-in electrode at its distal end is placed inside an elongate silicone tube to provide a catheter-like device. The core wire is manipulated at the proximal end of this arrangement by the physician during implantation to screw the helical electrode into heart tissue and fix the lead in place.

Unfortunately, silicone rubber has a tacky surface with high surface friction making assembly of internal components into silicone lumens during manufacture of pacemaker and cardiac leads difficult. For example, inserting the spiral conductor into the tubing is difficult because it tends to fasten to the silicone tubing. Also, this surface adherence impedes motion of parts making the extension of a helix in active-fix leads more difficult. Torque transfer through the tubing is difficult making difficult the turning of the core wire to screw the helical electrode into tissue. Further, due to sticking of the core wire to the inside of the tubing, flex life is shortened.

Previous practices to ameliorate these friction characteristics have involved; 1) the use of harder materials which are more slippery but less bio stable and less suitable for implantation e.g., polyurethane, 2) coating, 3) hardening, 4) swelling and, even 5) the use of environmentally unfriendly materials such as chlorofluorocarbons (CFC). Also, plasma discharge has been used on tubing with some degree of success. However, none of these practices have been satisfactory with respect to long lengths of narrow tubing and the provision of a uniform surface therein.

Another problem of implantable articles is how to obtain surface characteristics promoting growth of tissue on the surface to securely fastened the article after implantation.

US 5 830 329 describes a process of reducing the friction inside a small diameter tubing by placing the tubing in a close fitting glow discharge chamber with the plasma discharge gas inside the tubing and submitting the inner surface to glow discharge.

US 6 438 425 concerns another solution to the problem of reducing the coefficient of friction. In this case a tubular lead body is extruded to define a plurality of small parallel longitudinally extending grooves on the inner or outer surface of the lead body. The grooves may be formed by means of a die having inwardly or outwardly directed projections.

Presently, the assembly of silicone insulated leads is facilitated by using solvents such as isopropyl alcohol as lubiricant or n-heptane in order to temporarily swell the tubing to larger dimensions. Also, the silicone lumens are over-dimensioned to provide some extra mobility of internal parts. However, shrinkage after heptane-swelling is nonuniform and the tubing fastens to the conductors in localized places leaving tensions in the materials which are not easy to relax because of the adherence of silicone to the internal parts. Alternatively, relaxation after evaporation of isopropyl alcohol is impeded. Helix extension is also adversely affected by this adherence.

Another way to deal with the problems of assembly of silicone insulated leads is to over-dimension the silicone lumens to provide some extra mobility of internal parts. However, a general goal is to reduce dimensions, not to increase them.

The present methods do not provide adequate solutions to achieve easy assembly of pacemaker and cardiac leads.

### OBJECTS OF THE INVENTION

An object of the invention is to enable easy assembly of pacemaker and cardiac leads.

Another object is to provide an implantable elastomeric tubing having reduced surface friction on the inner or outer surface or on both surfaces.

A further object is to provide an implantable elastomeric article having a surface which promotes growth of tissue on the article after implantation.

An additional object is to provide an implantable elastomeric article having a surface roughness on at least one surface.

A further object is to provide a method of modifying at least one surface of implantable articles, such as tubing.

### BRIEF DESCRIPTION OF THE INVENTION

The invention concerns an implantable elastomeric article such as tubing with modified surface characteristics on at least one surface obtained by treatment with an acid.

The invention also concerns a method of treating at least one surface on an implantable elastomeric article with an acid to modified surface characteristics.

Further the invention concerns a method of assembly of an implantable electrode lead made of silicone tubing and a spiral conductor, comprising treating the tubing with an acid before inserting the conductor into the tubing.

### BRIEF DESCRIPTION OF THE DRAWINGS

Figure 1 shows attenuated total reflection-fourier transform infrared (ATR-FTIR) spectra for surface of untreated tubing and differently treated tubings.
Figure 2 shows optical microscope picture of untreated tubing.
Figures 3-5 show optical microscope pictures of treated tubings.

### DETAILED DESCRIPTION OF THE INVENTION

It was surprisingly found that the treatment of a surface of an implantable article of elastomeric material such as silicone provides a surface roughness which decreases the surface friction. The contact area between the spiral conductor and the silicone tubing during assembly of a pacemaker or cardiac lead is reduced owing to surface roughness obtained by treating the inside of the tubing with an acid. Thereby adherence between the spiral conductor and the lead is avoided and the insertion of the conductor facilitated. Further, it is possible to move the conductor inside the tubing and thus easier screw the electrode tip into heart tissue. A treatment of the outer surface of the silicone tubing may also result in the lead being more easily inserted into a blood vessel.

Thus, the surface roughness obtained by the treatment of an implantable article of elastomeric material with an acid gives a reduced friction between the article and another object. However, such surface roughness on an implantable article may also be of advantage to provide for growth of tissue on the article to fasten the implanted article.

Thus, the invention concerns an implantable elastomeric article having modified surface characteristics on at least one surface obtained by application of an acid on said at least one surface and after a predetermined treatment time removing the acid by rinsing.

Further, the invention concerns a method of modifying surface characteristics of at least one surface on an implantable elastomeric article, comprising application of an acid on said at least one surface and after a predetermined treatment time removing the acid by rinsing.

The invention also concerns a method of assembly of an implantable electrode lead including a spiral conductor inside a silicone tube having an inner surface and an outer surface, comprising application of an acid on said inner surface and after a predetermined treatment time removing the acid by rinsing with water followed by inserting the conductor into the tube.

The implantable elastomeric article of the invention may have a surface roughness of 0.1-50 µm on the treated surface.

The surface roughness is the medium depth of depressions in the surface or the height of the corresponding protrusions, from the base to the peak. The protrusions are formed in the surface of the elastomeric article by the treatment with an acid.

The height or depth may be evaluated optically with a visual microscope. A precision measurement may be made with an atomic force microscope, e.g. by Pacific Nanotechnology, Inc, Santa Clara, CA or Surface Science Laboratories, Sunnyvale, CA. A measurement may also be made using a laser beam to create a "Glitter Effect" as described by Bryan Beckingham and Professor Gregory Campbell, Department of Chemical Engineering, Clarkson University. Other methods comprise optical profilers, scatterometry and electron/ion beam methods.

The preferred implantable elastomeric material is silicone. The preferred acid for the treatment is hydrofluoric acid, HF. The following more detailed description is made in relation to silicone and treatment with hydrofluoric acid. However, this should not be interpreted as a limitation of the invention to these two materials.

The treatment with HF may be performed with a HF concentration of 0.02N - 20N. Preferably the concentration is between 0.1N and 10N and especially between 0.5 and 2N.

An appropriate treatment time would be at most 2 hours, corresponding to a minimum concentration of 0.1N. Rapid process less than 30 sec may be difficult to control. This gives a practical useful upper limit of 10N HF. A higher concentration could work faster if desired for a particular process. However, more rapid treatment leads to deeper and larger pitting before the entire surface is treated uniformly. Therefore, an optimum treatment is in the range of 0.5N - 2N HF for periods between 2.5 minutes and 5.5 minutes, giving the most uniform and finest grain surface roughness structures. Rougher structure may be desirable in some uses, though.

Treatment times and HF concentration are inversely proportional, to a fairly good approximation over the range of 0.02N - 20N HF.

The surface roughness can vary from 0.1 µm (width and depth) to 50 µm depending on treatment conditions. The surface roughness is suitably 0.2 to 20 µm, preferably 0.3 to 10 µm. Fine structures in the 0.5-2 µm range uniformly distributed over entire surface is optimum for effect in an implantable lead. A greater surface roughness may be used to achieve growth of tissue.

### EXAMPLES

Treatment of silicone tubing is carried out by contacting the silicone surfaces to be treated with 1.0 N (aq) hydrofluoric acid for 5.5 minutes. Contacting the inside of the lumen is facilitated by the use of a syringe to apply vacuum and suck the acid solution into the tubing. External surfaces are treated by immersion. Both internal and external surfaces can be treated simultaneously. Following the contact with acid, the silicone is flushed with distilled water. Then drying of the tubing (optional) is facilitated by an additional flush with isopropyl alcohol followed by blow-drying with pressurized (room temperature) air. The resulting treated surfaces have greater roughness (on microscopic scale), leading to less contact area with other components of the leads. There may or may not be some chemical modification of the surface as well, perhaps fluorination of the silicone polymer or deposition of lower-molecular weight silicone oils, which may contribute to the reduced adherence. In tubing with wall thickness as low as 0.12 mm treatment on both inside and outside of lumen gave a permanent (as far as we can tell at this time) improvement in handling and only reduced mechanical strength by about 5%.

### Practical treatment time limits and min/max HF concentration :

HF of concentrations of 0.02N - 20N were tested. Process time should be under 2 hrs to be most practical. Then 0.1N HF is minimum concentration. A rapid process less than 30 sec may also be difficult to control. Then 10N HF would be the practical useful upper limit - but higher concentration could work faster if desired for a particular process. However, more rapid treatment leads to deeper and larger pitting before entire surface is treated uniformly. Therefore, an optimum treatment is in the range of 0.5N - 2N HF for periods between 2.5 minutes and 5.5 minutes, giving the most uniform and finest grain surface roughness structures. Rougher structure may be desirable in some uses, though.

Treatment times and HF concentration are inversely proportional, to a fairly good approximation over the range of 0.02N - 20N HF.

### Size, topography and necessary wall thickness:

Visual microscope was used. The modified surface has roughness imparted by the HF treatment. This roughness can vary from 0.1 µm (width and depth) to 50 µm depending on treatment conditions (fine structures in the 0.5-2um range uniformly distributed over entire surface is optimum for effect in device). Should avoid so thin tubing that 50 µm is more than 20% of wall thickness. Above 50 µm surface feature size, the surface of the tubing obtains a wet feel, due to macroscopic amounts of unfilled silicone (PDMS) polymer at the surface -- unfilled PDMS has the undesirable feel and properties of a gel instead of a rubber.

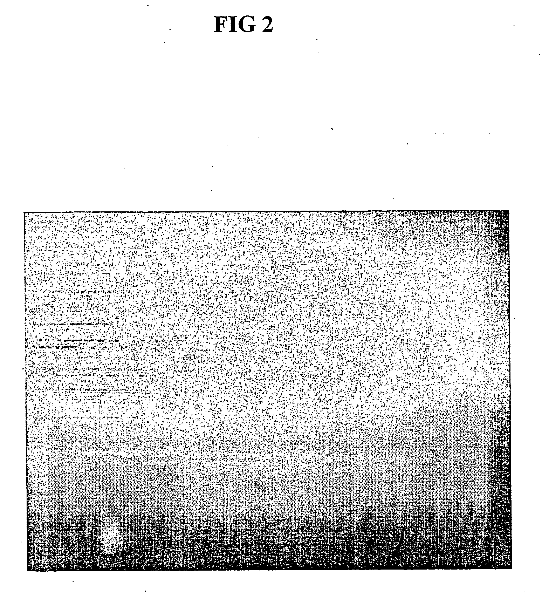

Fig 2 shows an optical microscope picture (400µm x 500µm) of untreated tubing (0.072" ID x 0.098" OD) MED4759 silicone rubber (extrusion lines clearly visible).

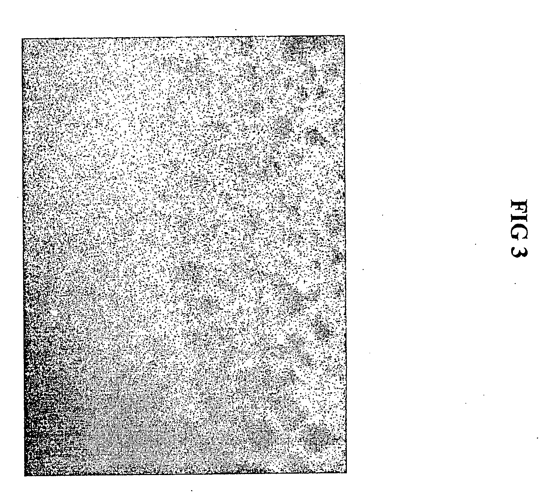

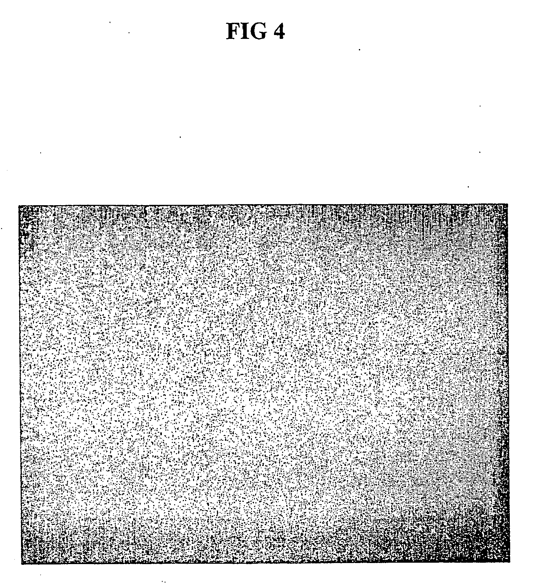

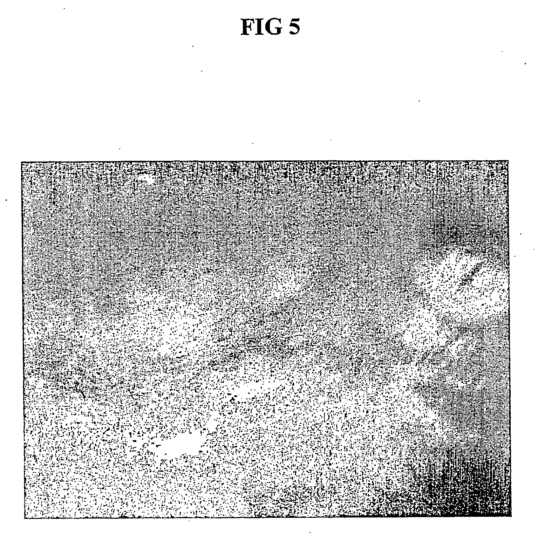

The result of different treatments are shown in Figs 3-5. Fig 3 being a microscope picture (400µm x 500µm): 20N HF for 44 sec (0.072" ID x 0.098" OD) MED4759 silicone rubber (deeper larger pitting). Fig 4 is an optical Microscope picture (400µm x 500µm): 2N HF for 2.5 min (0.072" ID x 0.098" OD) MED4759 silicone rubber (within optimum range). Fig 5 is an optical Microscope picture (400µm x 500µm): 2N HF for 54 min (0.072" ID x 0.098" OD) MED4759 silicone rubber (wet surface feel, exceeded optimum).

### Is F present after washing:

ATR-FTIR (surface measurement of infrared spectroscopy) shows that there is no chemical alteration of the PDMS polymer; no detectable fluorination occurs. The Si-O-Si stretching at 1020 cm-1 decreases in intensity in relation to the Si-CH₃ bending at 1259 cm⁻¹, indicating that the filler to polymer ratio at the surface is decreased in the HF-treated tubing. The decrease in filler content in the surface of treated tubing is greater for harsher treatments: for longer treatments at the same concentration or for more concentrated HF for a fixed time. This is consistent with what is known about HF that it is capable of corroding silicates (glass).

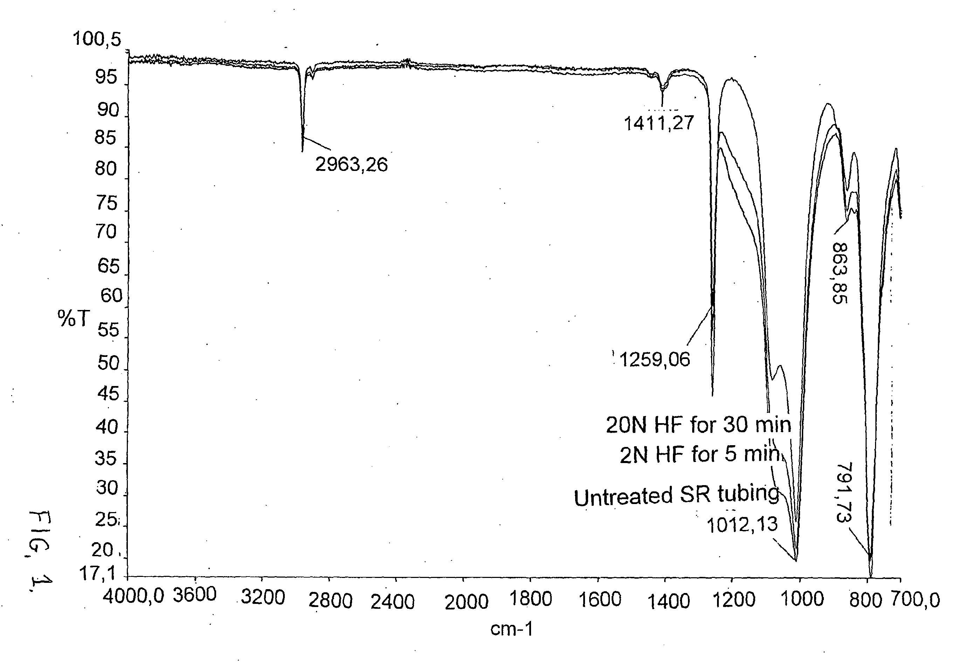

The result is shown on Fig 1 comprising ATR-FTIR spectra for surface of untreated tubing, optimal treated tubing, and excessively treated tubing (superimposed). This shows that no chemical modification such as fluorination or oxidation is occurring, and indicates that the HF treatment reduces the filler:polymer ratio at the surface.

### Number of turns/torque reduction:

A silicone tubing with surface modification according to the invention gives a 25% reduction in the amount of torque required to extend a helix on an active fixation lead from 0.8 mNm to 0.6 mNm.
The treated tubing also shows 12% reduction in moment (number of turns) from 8 to 7 on helix extension and 33% reduction in moment (number of turns) from 9 to 6 on helix retraction.

The invention is described above in the examples with reference to an implantable silicone tube. However, this should not be interpreted as a limitation of the scope of the invention. Modifications of both the material in the implantable article, the form of the article and the acid will be obvious to a person skilled in the art. The scope of the invention is defined in the enclosed claims.

## Claims

1. Implantable elastomeric article having on at least one surface a surface roughness resulting in reduced friction and obtained by application of an acid on said at least one surface and after a predetermined treatment time removing the acid by rinsing.

2. Implantable elastomeric article according to claim 1 which is made of silicone.

3. Implantable elastomeric article according to claim 1 or 2, wherein the acid used is hydrofluoric acid.

4. Implantable elastomeric article according to claim 3 wherein the concentration of the acid is between 0.1N and 10N, preferably between 0.2 and 5N and especially between 0.5 and 2N HF.

5. Implantable elastomeric article according to claim 4 wherein the treatment time is at most 2 hrs, preferably at most 1 h and especially at most 20 min.

6. Implantable elastomeric article according to claim 5 wherein the treatment time is at least 30 sec, preferably at least 1 min.

7. Implantable elastomeric article according to claim 4 wherein the treatment time is between 2 and 10 min, preferably between 2.5 and 5.5 min.

8. Implantable elastomeric article according to any of the earlier claims wherein the article is silicone tubing intended for pacemaker or cardiac leads, the tubing comprising an inner surface and an outer surface, only the inner surface having been treated with an acid.

9. Implantable elastomeric article according to any of the earlier claims wherein the article is silicone tubing intended for pacemaker or cardiac leads, the tubing comprising an inner surface and an outer surface, both the inner surface and the outer surface having been treated with an acid.

10. A method of achieving a rough surface giving a reduced friction on at least one surface on an implantable elastomeric article, comprising application of an acid on said at least one surface and after a predetermined treatment time removing the acid by rinsing.

11. A method according to claim 10, where said at least one surface is rinsed with water.

12. A method according to claim 10 or 11, wherein the article is made of silicone.

13. A method according to any of claims 10-12, wherein the acid used is hydrofluoric acid.

14. A method according to claim 13, where the concentration of the acid is between 0.1N and 10N, preferably between 0.2 and 5N and especially between 0.5 and 2N hydrofluoric acid.

15. A method according to claim 13 or 14, where the treatment time is at most 2 hrs, preferably at most 1 h and especially at most 20 min.

16. A method according to claim 15, where the treatment time is at least 30 sec, preferably at least 1 min.

17. A method according to claim 13 or 14, where the treatment time is between 2 and 10 min, preferably between 2.5 and 5.5 min.

18. A method according to any of claims 10-17, wherein the article is silicone tubing intended for pacemaker or cardiac leads, the tubing comprising an inner surface and an outer surface, the inner surface of the tubing being treated with an acid.

19. A method according to claim 18, where both the inner surface and the outer surface of the tubing are treated with an acid.

20. A method of assembly of an implantable electrode lead including a spiral conductor inside silicone tubing having an inner surface and an outer surface, comprising application of an acid on said inner surface and after a predetermined treatment time removing the acid by rinsing with water followed by inserting the conductor into the tubing.

21. A method of assembly according to claim 20, where the acid used is hydrofluoric acid.

22. A method of assembly according to claim 21, where the concentration of the acid is between 0.1N and 10N, preferably between 0.2 and 5N and especially between 0.5 and 2N hydrofluoric acid.

23. A method according to claim 20 or 21, where the treatment time is at most 2 hrs, preferably at most 1 h and especially at most 20 min.

24. A method according to claim 23, where the treatment time is at least 30 sec, preferably at least 1 min.

25. A method according to claim 21 or 22, where the treatment time is between 2 and 10 min, preferably between 2.5 and 5.5 min.

## Patentansprüche

1. Implantierbarer elastomerer Gegenstand, der auf wenigstens einer Fläche eine Oberflächenrauigkeit aufweist, die eine reduzierte Reibung zur Folge hat und durch Aufbringen einer Säure auf die genannte wenigstens eine Fläche und Entfernen der Säure nach einer vorbestimmten Behandlungszeit mittels Spülung erhalten worden ist.

2. Implantierbarer elastomerer Gegenstand nach Anspruch 1, der aus Silikon hergestellt ist.

3. Implantierbarer elastomerer Gegenstand nach Anspruch 1 oder 2, wobei die benutzte Säure eine Fluorwasserstoffsäure ist.

4. Implantierbarer elastomerer Gegenstand nach Anspruch 3, wobei die Konzentration der Säure zwischen 0,1N und 10N, vorzugsweise zwischen 0,2 und 5N und insbesondere zwischen 0,5 und 2N HF liegt.

5. Implantierbarer elastomerer Gegenstand nach Anspruch 4, wobei die Behandlungszeit höchstens 2 Stunden, vorzugsweise höchstens 1 Stunde und insbesondere höchstens 20 Minuten beträgt.

6. Implantierbarer elastomerer Gegenstand nach Anspruch 5, wobei die Behandlungszeit wenigstens 30 Sekunden, vorzugsweise wenigstens 1 Minute beträgt.

7. Implantierbarer elastomerer Gegenstand nach Anspruch 4, wobei die Behandlungszeit zwischen 2 und 10 Minuten, vorzugsweise zwischen 2,5 und 5,5 Minuten liegt.

8. Implantierbarer elastomerer Gegenstand nach einem der vorhergehenden Ansprüche, wobei der Gegenstand ein für einen Schrittmacher oder eine kardiale Leitung vorgesehener Silikonschlauch ist, der Schlauch eine Innenfläche und eine Außenfläche aufweist und nur die Innenfläche mit einer Säure behandelt worden ist.

9. Implantierbarer elastomerer Gegenstand nach einem der vorhergehenden Ansprüche, wobei der Gegenstand ein für einen Schrittmacher oder für kardiale Leitungen vorgesehener Silikonschlauch ist, der Schlauch eine Innenfläche und eine Außenfläche aufweist und sowohl die Innenfläche als auch die Außenfläche mit einer Säure behandelt worden sind.

10. Verfahren zum Erzielen einer rauen Fläche, das an wenigstens einer Fläche eines implantierbaren elastomeren Gegenstandes eine reduzierte Reibung ergibt, **gekennzeichnet durch** Aufbringen einer Säure auf die genannte wenigstens eine Fläche und Entfernen der Säure nach einer vorbestimmten Behandlungszeit **durch** Spülung.

11. Verfahren nach Anspruch 10, bei dem wenigstens eine Oberfläche mittels Wasser gespült wird.

12. Verfahren nach Anspruch 10 oder 11, bei dem der Gegenstand aus Silikon hergestellt ist.

13. Verfahren nach einem der Ansprüche 10 bis 12, bei dem die benutzte Säure eine Fluorwasserstoffsäure ist.

14. Verfahren nach Anspruch 13, bei dem die Konzentration der Säure zwischen 0,1N und 10N, vorzugsweise zwischen 0,2 und 5N und insbesondere zwischen 0,5 und 2N Fluorwasserstoffsäure liegt.

15. Verfahren nach Anspruch 13 oder 14, bei dem die Behandlungszeit höchstens 2 Stunden, vorzugsweise höchstens 1 Stunde und insbesondere höchstens 20 Minuten beträgt.

16. Verfahren nach Anspruch 15, bei dem die Behandlungszeit wenigstens 30 Sekunden, vorzugsweise wenigstens 1 Minute beträgt.

17. Verfahren nach Anspruch 13 oder 14, bei dem die Behandlungszeit zwischen 2 und 10 Minuten, vorzugsweise zwischen 2,5 und 5,5 Minuten liegt.

18. Verfahren nach einem der Ansprüche 10 bis 17, bei dem der Gegenstand ein für einen Schrittmacher oder für kardiale Leitungen vorgesehener Silikonschlauch ist, der Schlauch eine Innenfläche und eine Außenfläche aufweist und die Innenfläche des Schlauches mit einer Säure behandelt wird.

19. Verfahren nach Anspruch 18, bei dem sowohl die Innenfläche als auch die Außenfläche des Schlauches mit einer Säure behandelt werden.

20. Verfahren zur Montage einer implantierbaren Elektrodenleitung mit einem wendelförmigen Leiter innerhalb eines eine Innenfläche und eine Außenfläche aufweisenden Silikonschlauches, **gekennzeichnet durch** Aufbringen einer Säure auf die genannte Innenfläche und Entfernen der Säure **durch** Spülen mit Wasser nach einer vorbestimmten Behandlungszeit woraufhin das Einsetzen des Leiters in den Schlauch folgt.

21. Verfahren zur Montage nach Anspruch 20, bei dem die benutzte Säure Fluorwasserstoffsäure ist.

22. Verfahren zur Montage nach Anspruch 21, bei dem die Konzentration der Säure zwischen 0,1N und 10N, vorzugsweise zwischen 0,2 und 5N und insbesondere zwischen 0,5 und 2N Fluorwasserstoffsäure liegt.

23. Verfahren nach Anspruch 20 oder 21, bei dem die Behandlungszeit höchstens 2 Stunden, vorzugsweise höchstens 1 Stunde und insbesondere höchstens 20 Minuten beträgt.

24. Verfahren nach Anspruch 23, bei dem die Behandlungszeit wenigstens 30 Sekunden, vorzugsweise wenigstens 1 Minute beträgt.

25. Verfahren nach Anspruch 21 oder 22, bei dem die Behandlungszeit zwischen 2 und 10 Minuten, vorzugsweise zwischen 2,5 und 5,5 Minuten liegt.

## Revendications

1. Article élastomère implantable, ayant sur au moins une surface une rugosité de surface donnant un frottement réduit et obtenue par application d'un acide sur ladite au moins une surface et en enlevant, après une durée de traitement déterminée à l'avance, l'acide par rinçage.

2. Article élastomère implantable suivant la revendication 1 qui est en silicone.

3. Article élastomère implantable suivant la revendication 1 ou 2, dans lequel l'acide utilisé est l'acide fluorhydrique.

4. Article élastomère implantable suivant la revendication 3, dans lequel la concentration de l'acide est comprise entre 0,1 N et 10 N, de préférence entre 0,2 et 5 N, et, en particulier, entre 0,5 et 2 N pour HF.

5. Article élastomère implantable suivant la revendication 4, dans lequel la durée de traitement est au plus de 2 heures, de préférence au plus de 1 heure et, en particulier, au plus de 20 mn.

6. Article élastomère implantable suivant la revendication 5, dans lequel la durée de traitement est d'au moins 30 sec, de préférence d'au moins 1 mn.

7. Article élastomère implantable suivant la revendication 4, dans lequel la durée de traitement est comprise entre 2 et 10 mn, de préférence entre 2,5 et 5,5 mn.

8. Article élastomère implantable suivant l'une quelconque des revendications précédentes, dans lequel l'article est un tube en silicone destiné à des stimulateurs cardiaques ou à des dérivations cardiaques, le tube comprenant une surface intérieure et une surface extérieure, seule la surface intérieure ayant été traitée par un acide.

9. Article élastomère implantable suivant l'une quelconque des revendications précédentes, dans lequel l'article est un tube en silicone destiné à des stimulateurs cardiaques ou à des dérivations cardiaques, le tube comprenant une surface intérieure et une surface extérieure, à la fois la surface intérieure et la surface extérieure ayant été traitées par un acide.

10. Procédé d'obtention d'une surface rugueuse donnant un frottement réduit sur au moins une surface d'un article élastomère implantable, comprenant l'application d'un acide sur la au moins une surface et l'enlèvement, après une durée de traitement déterminée à l'avance, de l'acide par rinçage.

11. Procédé suivant la revendication 10, dans lequel on rince la au moins une surface à l'eau.

12. Procédé suivant la revendication 10 ou 11, dans lequel l'article est en silicone.

13. Procédé suivant l'une quelconque des revendications 10 à 12, dans lequel l'acide utilisé est l'acide fluorhydrique.

14. Procédé suivant la revendication 13, dans lequel la concentration de l'acide est comprise entre 0,1 N et 10 N, de préférence entre 0,2 et 5 N, et, en particulier, entre 0,5 et 2 N pour HF.

15. Procédé suivant la revendication 13 ou 14, dans lequel la durée de traitement est au plus de 2 heures, de préférence au plus de 1 heure et, en particulier, au plus de 20 mn.

16. Procédé suivant la revendication 15, dans lequel la durée de traitement est d'au moins 30 sec, de préférence d'au moins 1 mn.

17. Procédé suivant la revendication 13 ou 14, dans lequel la durée de traitement est comprise entre 2 et 10 mn, de préférence entre 2,5 et 5,5 mn.

18. Procédé suivant l'une quelconque des revendications 10 à 17, dans lequel l'article est un tube en silicone destiné à des stimulateurs cardiaques ou à des dérivations cardiaques, le tube comprenant une surface intérieure et une surface extérieure, seule la surface intérieure ayant été traitée par un acide.

19. Procédé suivant la revendication 18, dans lequel on traite à la fois la surface intérieure et la surface extérieure du tube par un acide.

20. Procédé d'assemblage d'une dérivation implantable d'électrode, comprenant un conducteur en spirale à l'intérieur d'un tube en silicone ayant une surface intérieure et une surface extérieure, comprenant l'application d'un acide sur la surface intérieure et, après une durée de traitement déterminée à l'avance, l'enlèvement de l'acide par rinçage à l'eau, suivi de l'insertion du conducteur dans le tube.

21. Procédé d'assemblage suivant la revendication 20, dans lequel l'acide utilisé est l'acide fluorhydrique.

22. Procédé d'assemblage suivant la revendication 21, dans lequel la concentration de l'acide est comprise entre 0,1 N et 10 N, de préférence entre 0,2 et 5 N, et, en particulier, entre 0,5 et 2 N pour HF.

23. Procédé d'assemblage suivant la revendication 20 ou 21, dans lequel la durée de traitement est au plus de 2 heures, de préférence au plus de 1 heure et, en particulier, au plus de 20 mn.

24. Procédé d'assemblage suivant la revendication 23, dans lequel la durée de traitement est d'au moins 30 sec, de préférence d'au moins 1 mn.

25. Procédé d'assemblage suivant la revendication 21 ou 22, dans lequel la durée de traitement est comprise entre 2 et 10 mn, de préférence entre 2,5 et 5,5 mn.
